Europäisches Patentamt

European Patent Office

Office européen des brevets

(19)

(11) **EP 0 589 488 B1**

(12) **EUROPÄISCHE PATENTSCHRIFT**

(45) Veröffentlichungstag und Bekanntmachung des Hinweises auf die Patenterteilung:
**17.09.1997 Patentblatt 1997/38**

(51) Int. Cl.$^6$: **C07C 255/59**, A61K 31/275, A23K 1/16

(21) Anmeldenummer: 93117935.2

(22) Anmeldetag: 26.04.1991

(54) **Im wesentlichen optisch reines (-)-Cimaterol und dessen Säureadditionssalze sowie deren Verwendung als Arzneimittel oder Leistungsförder**

Essentially optically pure (-)-Cimaterol and its addition salts as well as its use as a medicament or growth promotor

(-)-Cimaterol sensiblement pure et ses sels d'addition ainsi que son usage comme médicament ou comme promoteur de croissance

(84) Benannte Vertragsstaaten:
**AT BE CH DE DK ES FR GB GR IT LI LU NL SE**

(30) Priorität: **04.05.1990 DE 4014252**

(43) Veröffentlichungstag der Anmeldung:
**30.03.1994 Patentblatt 1994/13**

(62) Dokumentnummer(n) der früheren Anmeldung(en) nach Art. 76 EPÜ:
**91106774.2 / 0 455 155**

(73) Patentinhaber:
**BOEHRINGER INGELHEIM VETMEDICA GMBH
55216 Ingelheim (DE)**

(72) Erfinder:
• **Resemann, Wolfgang, Dr. Dipl.-Chem.
D-88400 Biberach (DE)**
• **Dürr, Adolf
D-88400 Biberach (DE)**
• **Engelhardt, Günther, Prof. Dr.
D-88400 Biberach (DE)**
• **Quirke, John Francis, Dr.
D-55611 Bingen (DE)**

(56) Entgegenhaltungen:
DE-B- 2 212 600      US-A- 4 119 710
US-A- 4 407 819

• **Goodman and Gilman's: The Pharmacological Basis of Therapeutics (8th Ed.) p.206**

**Beschreibung**

In der US-A-4.119.710 wird u. a. das Racemat 1-(4'-Amino-3'-cyano-phenyl)-2-isopropylamino-ethanol (generic name: Cimaterol) und dessen pharmazeutische Eigenschaften beschrieben. So weisen die in der US-A-4.119.710 beschriebenen Verbindungen neben einer analgetischen, uterusspasmolytischen und einer antispastischen Wirkung auf die quergestreifte Muskulatur, insbesondere $\beta_2$-mimetische und/oder $\beta_1$-blockierende Wirkungen auf, wobei je nach ihrer Substitution die eine oder andere Wirkung im Vordergrund steht. Außerdem wird in der US-A-4.119.710 festgestellt, daß die d-(+)-Verbindungen insbesondere eine selektive Wirkung auf die $\beta_1$-Rezeptoren und die 1-(-)-Verbindungen eine bevorzugte Wirkung auf die $\beta_2$-Rezeptoren aufweisen.

Desweiteren wird in der US-A-4.407.819 u. a. die leistungsfördernde Wirkung von Cimaterol bei Tieren beschrieben.

Es ist ferner bekannt, daß normalerweise eines der beiden Enantiomeren eines Racemats eine größere Wirksamkeit als das andere Enantiomere aufweist. Aus diesem Grunde lag der vorliegenden Anmeldung die Aufgabe zugrunde, das literaturbekannte Racemat in seine neuen Enantiomeren aufzutrennen.

Bei der Lösung dieser Aufgabe traten überraschenderweise Schwierigkeiten auf, obwohl bereits in der US-A-4.119.710 zwei Verfahren zur Racemattrennung dieser Verbindungen beschrieben werden, nämlich

a) die Auftrennung eine Gemisches von diastereomeren Verbindungen, die durch Umsetzung eines entsprechenden Racemats mit einem chiralen Acylrest erhalten werden, und anschließende Abspaltung des chiralen Acylrestes, z.B. des (-)-Menthylcarbonylrestes, oder
b) die fraktionierte Kristallisation eines Gemisches ihrer diastereomeren Salze mit einer optisch aktiven Hilfssäure.

Die Racemattrennung a) erschien dabei von Anfang an wegen der im Cimaterol vorhandenen Cyanogruppe wenig erfolgsversprechend zu sein, da bei der anschließend erforderlichen hydrolytischen oder hydrogenolytischen Abspaltung des chiralen Acylrestes die vorhandene Cyanogruppe zumindest teilweise hydrolytisch oder hydrogenolytisch verändert wird.

Die übliche Racemattrennung b), welche darin besteht, daß ein entsprechendes diastereomeres Salz durch Erhitzen in einem geeigneten Lösungsmittel gelöst und anschließend beim Abkühlen das schwerer lösliche diastereomere Salz zuerst auskristallisiert, wobei dieser Vorgang bis zur Isolierung des reinen schwerer löslichen diastereomeren Salzes erforderlichenfalls mehrmals wiederholt werden kann, und anschließend aus dem so erhaltenen reinen diastereomeren Salz die gewünschte enantiomere Base freigesetzt wird, führte überraschenderweise bei Cimaterol nicht zum Ziele, wie aus dem beigefügten Referenzbeispiel hervorgeht.

Überraschenderweise wurde nun gefunden, daß sich racemisches Cimaterol, das bereits eines der gewünschten Enantiomere in angereicherter Form enthalten kann, durch Lösen von Cimaterol und mindestens 2 Äquivalenten, zweckmäßigerweise von 2 bis 7 Äquivalenten, vorzugsweise jedoch von 2 bis 4 Äquivalenten, einer optisch aktiven zweibasischen Hilfssäure wie (-)-0,0'-Dibenzoyl-L-weinsäure, (+)-0,0'-Dibenzoyl-D-weinsäure, (-)-0,0'-Ditolyl-L-weinsäure oder (+)-0,0'-Ditolyl-D-weinsäure unter Einhaltung eines für jede Hilfssäure bestimmten Temperaturbereichs, welcher zweckmäßigerweise oberhalb von 20°C, vorzugsweise jedoch zwischen 25 und 30°C, liegt, in einem geeigneten Lösungsmittel über seine beiden diastereomeren Salze und anschließende Freisetzung der enantiomeren Base auftrennen läßt. Hierbei fällt das gewünschte diastereomere Salz mit einer Reinheit von mindestens ee = 90 % nach kurzer Zeit aus, z. B. nach 1 bis 5 Minuten, wobei allerdings der für jede Hilfssäure charakteristische Temperaturbereich, welcher durch übliche Handversuche ermittelt werden kann, nicht unterschritten werden darf. Die Lösung der beiden Komponenten erfolgt hierzu vorzugsweise gleichzeitig, eine Lösung der einen Komponenten kann selbstverständlich auch zu einer Lösung der anderen Komponenten gegeben werden.

Besonders vorteilhaft wird die Racemattrennung mit 2 bis 7 Äquivalenten, vorzugsweise mit 1 bis 3 Äquivalenten von (-)-0,0'-Dibenzoyl-L-weinsäure oder (+)-0,0'-Dibenzoyl-D-weinsäure als Hilfssäure bei Temperaturen oberhalb von 25°C, vorzugsweise jedoch in einem Temperaturbereich zwischen 25 und 30°C, durchgeführt. So erhält man beispielsweise nach gleichzeitigem Lösen von 1 Mol Cimaterol und 1 Mol (-)-0,0'-Dibenzoyl-L-weinsäure oder (+)-0,0'-Dibenzoyl-D-weinsäure in Methanol jeweils reines (+)-1-(4'-Amino-3'-cyano-phenyl)-2-isopropylamino-ethanol-(-)-0,0'-dibenzoyl-L-hydrogentartrat oder (-)-1-(4'-Amino-3'-cyano-phenyl)-2-isopropylamino-ethanol-(+)-0,0'-dibenzoyl-D-hydrogentartrat, wobei nach kurzer Zeit, z. B. nach 1 bis 2 Minuten, jeweils das diesbezügliche reine diastereomere Hydrogentartrat zu kristallisieren beginnt. Dieses Salz fällt schon nach dem ersten Schritt in einer hohen Reinheit von mindestens ee = 94 % an. Sollte eine noch größere Reinheit erforderlich sein, so kann durch Lösen des nach einmaliger Fällung erhaltenen diastereomeren Hydrogentartrats und erneutes Kristallisieren oberhalb von 25°C das Trennverfahren einmal oder erforderlichenfalls mehrmals wiederholt werden.

Von dem so erhaltenen diastereomeren Salz wird anschließend mittels einer Base, vorzugsweise mittels verdünntem Ammoniak, die gewünschte im wesentlichen optisch reine enantiomere Base freigesetzt und nach üblichen Methoden isoliert.

Die erhaltenen enantiomeren Basen können gewünschtenfalls anschließend in ihre Säureadditionssalze, insbesondere für die pharmazeutische Anwendung in ihre physiologisch verträglichen Salze mit anorganischen oder organischen Säuren, vorzugsweise jedoch mit organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Besonders bemerkenswert ist jedoch, daß bei einer versehentlichen Unterschreitung des Temperaturbereiches das beispielsweise erhaltene racemische Cimaterol-0,0'-dibenzoyl-L- oder Cimaterol-0,0'-dibenzoyl-D-hydrogentartrat nach erneutem Aufschlämmen und Erwärmen über 25°C, in das gewünschte reine diastereomere Hydrogentartrat übergeführt werden kann.

Gegenstand der vorliegenden Erfindung ist somit (-)-Cimaterol, das im wesentlichen optisch rein ist, beispielsweise eine optische Reinheit von mindestens ee = 90 %, vorzugsweise von 94 bis 100 %, aufweist, dessen Säureadditionssalze, insbesondere dessen physiologisch verträgliche Säureadditionssalze mit anorganischen oder organischen Säuren, die Verwendung von im wesentlichen optisch reinem (-)-Cimaterol und dessen physiologisch verträgliche Säureadditionssalze als Arzneimittel und Leistungsförderer bei Tieren.

Aus dem nachfolgenden Versuchsbericht geht hervor, daß das L-Enantiomere von Cimaterol, also (-)-Cimaterol, wobei unter (-)-Cimaterol dasjenige Enantiomere zu verstehen ist, das die Schwingungsebene von linearpolarisiertem Licht der Natrium-D-Linie nach links dreht, überraschenderweise der alleinige Träger der biologischen Wirkungen von Cimaterol ist:

1. Die beiden Enantiomeren von Cimaterol wurden vergleichend auf eine β-mimetische Wirkung an der glatten Muskulatur des Bronchus, der Skelettmuskulatur und den Fettzellen untersucht. Darüberhinaus wurde ihre akute Toxizität verglichen.

Die Prüfung der β-mimetischen Wirkung an der glatten Muskulatur erfolgte als broncholytische Wirkung in der Versuchsanordnung nach KONZETT u. RÖSSLER (Arch. Exp. Path. Pharmakol. 195, 71-74 (1940)) gegenüber dem Acetylcholin-bedingten Bronchospasmus des Meerschweinchens nach i.v.-Applikation. Aus der mit den verschiedenen Dosen der Prüfsubstanz erzielten Hemmung des Bronchospasmus wurde nach linearer Regressionsanalyse eine $ED_{50}$ als jene Dosis berechnet, die zu einer 50%igen Hemmung des Bronchospasmus führte.

Die nachfolgende Tabelle enthält die gefundenen Werte:

| Substanz | $ED_{50}$ μg/kg |
|---|---|
| (-)-Cimaterol | 0,27 (0,18 - 0,38) |
| (+)-Cimaterol | 31,30 (5,50 - 48,30) |

Das L-Enantiomere ist an den β-Rezeptoren des Bronchus etwa 100 mal wirksamer als das entsprechende D-Enantiomere.

Die Prüfung der β-mimetischen Wirkung an der Skelettmuskulatur erfolgte mit Hilfe der Methode von BOWMAN a. NOTT (Pharmacol. Rev. 21, 27 (1969)). Es wurde der Einfluß auf die Spannung des sich inkomplett tetanisch kontrahierenden M. soleus von narkotisierten Katzen nach i.v.-Applikation geprüft.

Die dabei erzielten Resultate finden sich in der nachfolgenden Tabelle:

| Substanz | Dosis μg/kg | n | Abnahme der Spannung in % | |
|---|---|---|---|---|
| | | | x | SD |
| Cimaterol | 0,5 | 5 | 38,3 | 10,8 |
| (-)-Cimaterol | 0,5 | 5 | 42,1 | 6,0 |
| (+)-Cimaterol | 50,0 | 5 | 33,7 | 8,0 |

Das L-Enantiomere des Cimaterol ist auch an den β-Rezeptoren der Skelettmuskulatur mehr als 100 mal wirksamer als das entsprechende D-Enantiomere.

Die Prüfung der β-mimetischen Wirkung auf die Fettzellen erfolgte als lipolytische Wirkung an wachen Kaninchen nach i.v.-Applikation. Aus den mit den verschiedenen Dosen der Prüfsubstanzen erzielten Änderungen der freien Fettsäuren im Blut der Kaninchen (bestimmt mit der Methode nach DUNCOMBE: Biochem. J. 88, 7 (1963)) wurde nach linearer Regressionsanalyse eine $ED_{150}$ als jene Dosis berechnet, die zu einer 50%-igen Erhöhrung der freien Fettsäure im Blut führte.

Die hierbei erhobenen Befunde sind in der nachfolgenden Tabelle zusammengestellt:

| Substanz | $ED_{150}$ µg/kg |
|---|---|
| (-)-Cimaterol | 0,098 (0,078 - 0,118) |
| (+)-Cimaterol | 9,770 (7,440 - 12,700) |

Die zur Erzielung einer 50%igen Erhöhung der freien Fettsäuren im Blut des Kaninchens von D-Cimaterol benötigten Dosen liegen etwa 100 mal höher als die des entsprechenden L-Enantiomeren.

Die Bestimmung der akuten Toxizität erfolgte an Mäusen beider Geschlechter in einem Gewicht zwischen 20 und 25 g nach i.v.-Applikation. Aus dem Prozentsatz der Tiere, die innerhalb von 14 Tagen nach der Substanzgabe verstarben, wurde eine $LD_{50}$ nach Probit-Analyse berechnet.

Die nachfolgende Tabelle enthält die gefundenen Werte:

| Substanz | $LD_{50}$ mg/kg |
|---|---|
| (-)-Cimaterol | 74,8 (70,6 - 79,2) |
| (+)-Cimaterol | 86,3 (80,6 - 92,3) |

2. Die leistungsfördernde Wirkung von (-)-Cimaterol wurde an Gruppen von jeweils 10 acht Monate alten Lämmern (Suffolk x Galway wether lambs) untersucht, welche 6 Wochen lang mit einem Futter (Trockenzusammensetzung pro kg: 145 g Rohprotein + 70 g Rohfaser + 17 g Öl + 50 g Rohasche) gefüttert wurden, dem jeweils 2 mg/kg die zu untersuchende Substanz zugemischt war. Gleichzeitig wurde sichergestellt, daß den Lämmern während der Versuchsperiode mindestens 10 % Futterüberschuß angeboten wurde. Danach wurden die Lämmer geschlachtet und folgende Parameter ermittelt:

| | Kontrolle | (+)-Cimaterol | (-)-Cimaterol | Cimaterol |
|---|---|---|---|---|
| Anfangsgewicht in kg | 34.4 | 34.8 | 34.2 | 34.9 |
| Endgewicht in kg | 45.4 | 45.7 | 48.0 | 46.0 |
| Gewichtszunahme in g pro Tag | 259 | 268 | 324 | 275 |
| Futterverwertung in g Gewichts zunahme pro kg Futter | 190 | 184 | 220 | 196 |
| Gewicht des Schlachtkörpers in kg | 22.8 | 22.5 | 24.1 | 24.0 |
| Zusammensetzung des Schlachtkörpergewebes (ohne Knochen) in g pro kg: | | | 557 | 556 |
| Fett | 348 | 341 | 252 | 265 |
| Protein | 140 | 151 | 177 | 167 |

Wie bereits vorstehend erwähnt, geht aus den vorstehenden biologischen Daten hervor, daß überraschenderweise (-)-Cimaterol der alleinige Träger der erwünschten Wirkungen ist. Da das D-Enantiomere aber mit der gleichen Toxizität behaftet ist wie das L-Enantiomere, läßt sich durch den Einsatz des L-Cimaterols anstelle des Racemates das Nutzen/Risikoverhältnis um den Faktor 2 verbessern.

(-)-Cimaterol und dessen physiologisch verträglichen Säureadditionssalze eignet sich daher zur Behandlung der Fettsucht, von obstruktiven Lungenveränderungen, allergischem Asthma-Bronchiale, spastischen Bronchitiden, Entzündungen oder vorzeitiger Wehentätigkeit.

Beim Erwachsenen liegt hierbei die Einzeldosis zwischen 0,01 und 50 µg, vorzugsweise jedoch zwischen 0,01 und 10 µg, 2- bis 4-mal täglich. Hierzu läßt sich (-)-Cimaterol oder dessen physiologisch verträglichen Salze gegebenenfalls in Kombination mit anderen Wirksubstanzen, in die üblichen galenischen Zubereitungsformen wie Pulver, Tabletten, Dragées, Kapseln, Suppositorien oder Suspensionen einarbeiten.

Desweiteren kann (-)-Cimaterol und dessen Säureadditionssalze, wie bereits eingangs erwähnt, zur Behandlung fettsüchtiger Tiere wie Hunde und als Folge ihrer körperfettreduzierenden (lipolytischen) Wirkung zur Reduktion unerwünschter Fetteinlagerungen bei der Mastzucht, also zur Verbesserung der Fleischqualität von Masttieren wie Schweinen, Rindern, Schafen und Geflügel eingesetzt werden. Bei den Tieren kann die Applikation der oben genannten Verbindungen oral oder auch nicht-oral, z.B. als Futterzusatz oder durch Injektion oder auch durch Implantate erfolgen. Die Tagesdosis liegt hierbei zwischen 0,01 und 100 µg/kg, vorzugsweise jedoch zwischen 0,01 bis 10 µg/kg Körpergewicht.

Desweiteren kann (-)-Cimaterol und dessen Säureadditionssalze als Leistungsförderer bei Tieren zur Förderung und Beschleunigung des Wachstums, der Milch- und Wollproduktion, sowie zur Verbesserung der Futterverwertung, der Schlachtkörperqualität und zur Verschiebung des Fleisch-Fett-Verhältnisses zugunsten von Fleisch eingesetzt werden. Die Wirkstoffe werden bei Nutz-, Zucht-, Zier- und Hobbytieren verwendet.

Zu den Nutz- und Zuchttieren zählen Säugetiere wie z.B. Rinder, Schweine, Pferde, Schafe, Ziegen, Kaninchen, Hasen, Damwild, Pelztiere wie Nerze, Chinchilla, Geflügel wie z.B. Hühner, Gänse, Enten, Truthühner, Fische, wie z.B. Karpfen, Forellen, Lachse, Aale, Schleien, "yellow tails", Hechte, Reptilien wie z.B. Schlangen und Krokodile.

Zu den Zier- und Hobbytieren zählen Säugetiere wie Hunde und Katzen, Vögel wie Papageien, Kanarienvögel, Fische wie Zier- und Aquarienfische, z.B. Goldfische.

Die Wirkstoffe werden unabhängig vom Geschlecht der Tiere während allen Wachstums- und Leistungsphasen der Tiere eingesetzt. Bevorzugt werden die Wirkstoffe während der intensiven Wachstums- und Leistungsphase eingesetzt. Die intensive Wachstums- und Leistungsphase dauert je nach Tierart von einem Monat bis zu 10 Jahren.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,01 bis 50 µg/kg insbesondere 0,01 bis 25 µg/kg Körpergewicht pro Tag. Die passende Menge des Wirkstoffs sowie die passende Dauer der Verabreichung hängen insbesondere von der Tierart, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Tierart, dem Verhalten und dem Gesundheitszustand der Tiere ab.

Die Wirkstoffe können einmalig verabreicht werden. Die Wirkstoffe können aber auch während der ganzen oder während eines Teils der Wachstumsphase temporär oder kontinuierlich verabreicht werden. Bei kontinuierlicher Verabreichung kann die Anwendung ein- oder mehrmals täglich in regelmäßigen oder unregelmäßigen Abständen erfolgen.

Die Verabreichung erfolgt oral oder parenteral in dafür geeigneten Formulierungen oder in reiner Form. Orale Formulierungen sind Pulver, Tabletten, Granulate, Drenche, Boli sowie Futtermittel, Prämixe für Futtermittel, Formulierungen zur Verabreichung über Trinkwasser.

Die oralen Formulierungen enthalten den Wirkstoff in Konzentrationen von 0,01 ppb - 100 %, bevorzugt von 0,01 ppb - 10 %.

Parenterale Formulierungen sind Injektionen in Form von Lösungen, Emulsionen und Suspensionen, sowie Implantate.

Die Wirkstoffe können in den Formulierungen allein oder in Mischung mit anderen Wirkstoffen, Mineralsalzen, Spurenelementen, Vitaminen, Eiweißstoffen, Farbstoffen, Fetten oder Geschmacksstoffen vorliegen.

Die Konzentration der Wirkstoffe im Fertigfutter beträgt normalerweise etwa 0,01 ppb - 50 ppm, bevorzugt 0,1 ppb - 10 ppm.

Die Wirkstoffe können als solche oder in Form von Prämixen oder Futterkonzentraten dem Futter zugesetzt werden.

So enthalten die erfindungsgemäßen Futtermittel neben dem Wirkstoff und gegebenenfalls neben einer üblichen Vitamin-Mineral-Mischung beispielsweise für Mastschweine Gerste, Weizennachmehl, Ackerbohnen, Raps-Extraktionsschrot und Futterfett, für Broiler Mais, Sojabohnenmehl, Fleischmehl, Futterfett und Sojaöl, für Rinder Zuckerrübenschnitzel Maiskleber, Malzkeime, Sojabohnenmehl, Weizen und Molasse sowie für Lämmer Gerste, Sojabohnenmehl, Mais und Melasse. Zu diesem Futter wird eine der eingangs erwähnten Verbindungen der Formel I als Wirkstoff in einer Konzentration von 0,01 ppb bis 0,50 %, vorzugsweise jedoch von 0,1 ppb bis 0,05 %, zugemischt, wobei die Zumischung vorzugsweise in Form einer Wirkstoffvormischung erfolgt. Diese Vormischung enthält beispielsweise 5 bis 10 000 mg, vorzugsweise jedoch 50 bis 1 000 mg, zweckmäßigerweise in 1 000 g Maisstärke.

Die nachfolgenden Beispiele sollen die Erfindung näher erläutern:

Referenzbeispiel

(-)-1-(4'-Amino-3'-cyano-phenyl)-2-isopropylamino-ethanol

a) (-)-1-(4'-Amino-3'-cyano-phenyl)-2-isopropylamino-ethanol-(+)-0,0'-dibenzoyl-D-hydrogentartrat

50,0 g (0,23 Mol) Cimaterol und 82,4 g (0,23 Mol) (+)-0,0'-Dibenzoyl-D-weinsäure werden in 500 ml Methanol bei Raumtemperatur gelöst. Nach kurzer Zeit kristallisiert das Hydrogentartrat des Cimaterols aus. Die Kristallsuspension wird ca. 2 Stunden bei 19-20°C Innentemperatur gerührt, abgesaugt und der Filterrückstand mit kaltem Methanol nachgewaschen sowie bei 50°C bis zur Gewichtskonstanz getrocknet.

Ausbeute: 90 g (68,0 % der Theorie bezogen auf eingesetztes Racemat),
Schmelzpunkt der freigesetzten Base: 162,8°C
ee = 8

b) 88 g des gemäß a) erhaltenen Hydrogentartrats werden in 500 ml Methanol bis zur vollständigen Lösung unter Rückfluß gekocht und die klare Lösunge wieder auf ca. 15°C Kolbeninnentemperatur abgekühlt. Das ausfallende Kristallisat wird noch ca. 2-3 Stunden bei 15-18°C gerührt, abgesaugt, mit kaltem Methanol gewaschen und getrocknet.

Ausbeute: 62 g (70,5 % des Einsatzes),
Schmelzpunkt der freigesetzten Base: 160,9°C
ee = 24

c) 60 g des gemäß b) erhaltenen Hydrogentartrats werden in 350 ml Methanol analog a) in der Siedehitze gelöst, das Kristallisat ca. 12 Stunden bei 10-15°C gerührt, abgesaugt, mit kaltem Methanol gewaschen und getrocknet.

Ausbeute: 34 g (56,7 % des Einsatzes),
Schmelzpunkt der freigesetzten Base: 151,6°C
ee = 45

Nach der 2. Umkristallisation betrug die Gesamtausbeute nur noch 27,2 % der Theorie bezogen auf das eingesetzte Racemat, wobei nur ein enantimomerer Überschuß (ee) von 45 erzielt wurde. Wegen der großen Verluste wurde auf eine Fortführung der Kristallisation verzichtet.

Beispiel 1

(-)-1-(4'-Amino-3'-cyano-phenyl)-2-isopropylamino-ethanol

a) (-)-1-(4'-Amino-3'-cyano-phenyl)-2-isopropylamino-ethanol-(+)-0,0'-dibenzoyl-D-hydrogentartrat

50 g (0,23 Mol) 1-(4'-Amino-3'-cyano-phenyl)-2-isopropylamino-ethanol und 82,4 g (0,23 Mol) (+)-0,0'-Dibenzoyl-D-weinsäure werden in einem 1 l-Dreihalskolben in 500 ml Methanol bei 25 bis 28°C unter Rühren gelöst. Aus der klaren Lösung kristallisiert nach ca. 1 bis 2 Minuten das linksdrehende diastereomere Hydrogentartrat. Nach ca. 2 Stunden Nachrührzeit bei 25 bis 28°C wird das Kristallisat abgesaugt, mit wenig Methanol nachgewaschen und bei 50°C im Umlufttrockenschank getrocknet.

Ausbeute: 44 g (66,5 % der Theorie),
Schmelzpunkt: 140-141°C

b) 42 g des gemäß Beispiel 1a erhaltenen Hydrogentartrats werden in einem 1 l Dreihalskolben in der Siedehitze unter Rühren in 550 ml Methanol gelöst und die heiße Lösung in einer 2 l Filterpresse über ein Seitz-EK-Filter klarfiltriert. Das Filtrat wird unter schwachem Vakuum auf ca. 100 - 150 ml Lösung eingeengt und die entstandene Kristallsuspension ca. 1 Stunde bei 25 bis 28°C nachgerührt. Das Kristallisat wird abgesaugt, mit wenig Methanol nachgewaschen und bei 50°C im Umlufttrockenschrank getrocknet.

Ausbeute: 35,7 g (85 % bezogen auf das eingesetzte Hydrogentartrat),
Schmelzpunkt: 140-141°C

c) (-)-1-(4'-Amino-3'-cyano-phenyl)-2-isopropylamino-ethanol

36,0 g des gemäß Beispiel 1b erhaltenen Hydrogentartrats werden bei Raumtemperatur in 50 ml konzentriertem Ammoniak und 150 ml Wasser nach und nach eingetragen. Nach 1 Stunde Rühren wird die Kristallsuspension abfiltriert, gut mit deionisiertem Wasser nachgewaschen und bei Raumtemperatur im Umlufttrockenschrank bis zur Gewichtskonstanz getrocknet.

Ausbeute: 11,7 g (85,6 % der Theorie),
Schmelzpunkt: 146,5°C
$\alpha$ = - 4,37°
$[\alpha]_D^{20}$ = - 31°
ee = 99,4

Beispiel 2

(+)-1-(4'-Amino-3'-cyano-phenyl)-2-isopropylamino-ethanol

a) (+)-1-(4'-Amino-3'-cyano-phenyl)-2-isopropylamino-ethanol-(-)-0,0'-dibenzoyl-L-hydrogentartrat
Hergestellt aus 1-(4'-Amino-3'-cyano-phenyl)-2-isopropylamino-ethanol und (-)-0,0'-Dibenzoyl-L-weinsäure analog Beispiel 1a.

Ausbeute: 69,8 % der Theorie,
Schmelzpunkt: 140-141°C

b) Die weitere Reinigung erfolgt analog Beipiel 1b.

Ausbeute: 83 % bezogen auf das eingesetzte Hydrogentartrat,
Schmelzpunkt: 140-141°C

c) (+)-1-(4'-Amino-3'-cyano-phenyl)-2-isopropylamino-ethanol
Hergestellt analog Beispiel 1c.

Ausbeute: 81 % der Theorie,
Schmelzpunkt: 146,5°C
$\alpha$ = + 4,38°
$[\alpha]_D^{20}$ = + 31,5°, ee = 98,2

Beispiel 3

(+)-1-(4'-Amino-3'-cyano-phenyl)-2-isopropylamino-ethanol

Die aus der Fällung gemäß Beispiel 1a erhaltene Mutterlauge wird im Vakuum zur Trockene eingedampft und der erhaltene Rückstand mit 50 ml konzentriertem Ammoniak und 150 ml Wasser aufgenommen und bei ca. 5 bis 10°C 2 Stunden lang gerührt. Nach dem Absaugen wird der Filterrückstand mit Wasser gut gewaschen und im Umlufttrockenschrank bei Raumtemperatur gut getrocknet.

Ausbeute: 26,5 g (53 % bezogen auf eingesetztes Racemat)

Das so erhaltene rohe (+)-1-(4'-Amino-3'-cyano-phenyl)-2-isopropylamino-ethanol wird mit (-)-0,0'-Dibenzoyl-L-weinsäure analog Beispiel 2 gereinigt:

a)

Ausbeute: 39,7 g (56,8 % bezogen auf eingesetztes Racemat-Enantiomeren-Gemisch),
Schmelzpunkt: 140-141°C

b)

Ausbeute: 87 % bezogen auf das eingesetzte Hydrogentartrat,
Schmelzpunkt: 140-141°C

**EP 0 589 488 B1**

c)

Ausbeute: 78,5 % der Theorie,
Schmelzpunkt: 146,5°C
$\alpha = +4,37°$
$[\alpha]_D^{20} = +31°$, ee = 98

Beispiel 4

(-)-1-(4'-Amino-3'-cyano-phenyl)-2-isopropylamino-ethanol

50 g (-)-1-(4'-Amino-3'-cyano-phenyl)-2-isopropylamino-ethanol-(+)-0,0'-dibenzoyl-L-hydrogentartrat (hergestellt gemäß Referenzbeispiel a), Schmelzpunkt der freigesetzten Base: 162,8°C; ee = 8) werden mit 500 ml Methanol aufgeschlämmt und bei ca. 28°C Kolbeninnentemperatur eine Stunde gerührt. Anschließend wird abgesaugt, mit wenig Methanol gewaschen und bis zur Gewichtskonstanz bei 50°C getrocknet.

Ausbeute: 28,0 g (56 % bezogen auf Racemat-Enantiomeren-Gemisch).

Das so erhaltene Hydrogentartrat wird analog Beispiel 1c) mittels verdünntem Ammoniak freigesetzt.

Ausbeute: 9,14 g (86 % der Theorie),
Schmelzpunkt: 145°C
$\alpha = -4,37°$
$[\alpha]_D^{20} = -31°$
ee = 98

Beispiel 5

Tabletten mit 2 µg (-)-Cimaterol

Zusammensetzung:

1 Tablette enthält:

| Wirksubstanz | 0,002 mg |
|---|---|
| Milchzucker | 82,498 mg |
| Kartoffelstärke | 33,000 mg |
| Polyvinylpyrrolidon | 4,000 mg |
| Magnesiumstearat | 0,050 mg |
| | 120,000 mg |

Herstellungsverfahren:

Die Wirksubstanz und Polyvinylpyrrolidon werden in Äthanol gelöst. Die Mischung von Milchzucker und Kartoffelstärke wird mit der Wirkstoff-/Granulierlösung gleichmäßig befeuchtet. Die Feuchtsiebung erfolgt mit 1,5 mm-Maschenweite. Anschließend wird bei 50°C getrocknet und die Trockensiebung mit 1,0 mm-Maschenweite vorgenommen. Das so erhaltene Granulat wird mit Magnesiumstearat gemischt und zu Tabletten verpreßt.

8

| Tablettengewicht: | 120 mg |
|---|---|
| Stempel: | 7 mm, flach |

Beispiel 6

Dragées mit 1 µg (-)-Cimaterol

Zusammensetzung:

1 Tablette enthält:

| Wirksubstanz | 0,001 mg |
|---|---|
| Milchzucker | 82,499 mg |
| Kartoffelstärke | 33,000 mg |
| Polyvinylpyrrolidon | 4,000 mg |
| Magnesiumstearat | 0,500 mg |
| | 120,000 mg |

Herstellungsverfahren:

Dragéekerne analog Tabletten Beispiel 5.

| Kerngewicht: | 120 mg |
|---|---|
| Stempel: | 7 mm, gewölbt |

Die Kerne werden nach bekanntem Verfahren mit einer Hülle überzogen, die im wesentlichen aus Zucker und Talkum besteht. Die fertigen Dragées werden mit Bienenwachs poliert.

Dragéegewicht: 200,0 mg

Beispiel 7

Gelatine-Steckkapseln mit 1 µg (-)-Cimaterol

Zusammensetzung:

1 Tablette enthält:

| | |
|---|---|
| Wirksubstanz | 0,001 mg |
| Milchzucker | 59,999 mg |
| Maisstärke | 60,000 mg |
| | 120,000 mg |

Herstellungsverfahren:

Die Wirksubstanz wird mit Milchzucker und Maisstärke intensiv gemischt und in Gelatine-Steckkapseln geeigneter Größe abgefüllt.

Kapselfüllung: 120,0 mg

Beispiel 8

Ampullen mit 2 µg (-)-Cimaterol pro 2 ml

Zusammensetzung:

1 Ampulle enthält:

| | |
|---|---|
| Wirksubstanz | 0,002 mg |
| Zitronensäure | 2,500 mg |
| Natriumhydrogenphosphat | 7,500 mg |
| Kochsalz | 4,600 mg |
| Ampullenwasser ad | 2,000 ml |

Herstellungsverfahren:

Die Wirksubstanz, Puffersubstanzen und Kochsalz werden in Ampullenwasser gelöst und anschließend keimfrei filtriert.

Abfüllung: in braune Ampullen zu 2 ml unter Schutzbegasung ($N_2$)
Sterilisation: 20 Minuten bei 120°C

Beispiel 9

Suppositorien mit 2,5 µg (-)-Cimaterol

Zusammensetzung:

1 Zäpfchen enthält:

| Wirksubstanz | 0,025 mg |
|---|---|
| Suppositorienmasse (z.B. Witepsol W 45) | 1 699,975 mg |
| | 1 700,000 mg |

Herstellungsverfahren:

In die geschmolzene und auf 40°C abgekühlte Zäpfchenmasse wird die feinpulverisierte Wirksubstanz mit Hilfe eines Eintauchhomogenisators eingerührt und die Masse bei 37°C in leicht vorgekühlte Formen ausgegossen.

Zäpfchengewicht: 1,7 g

Beispiel 10

Sirup mit 2 µg (-)-Cimaterol pro 5 ml

Zusammensetzung:

100 ml Sirup enthält:

| Wirksubstanz | 0,04 mg |
|---|---|
| Benzoesäure | 0,10 g |
| Weinsäure | 1,00 g |
| Zucker | 50,00 g |
| Apfelsinen-Aroma | 1,00 g |
| Lebensmittelrot | 0,05 g |
| Dest. Wasser ad | 100,00 ml |

Herstellungsverfahren:

Ca. 60 g dest. Wasser werden auf 80°C erwärmt und darin nacheinander Benzoesäure, Weinsäure, die Wirksubstanz, der Farbstoff und Zucker gelöst. Nach Abkühlung auf Raumtemperatur wird das Aroma zugegeben und auf das gegebene Volumen aufgefüllt. Der Sirup wird filtriert.

Beispiel 11

Aerosol-Spray mit 1 µg (-)-Cimaterol pro Hub

Zusammensetzung:

| Wirksubstanz | 0,00025 mg |
|---|---|
| Soja-Lecithin | 0,05000 mg |
| Treibgasgemisch 11/12/114 (23:54:23) | 69,94975 mg |
| | 70,00000 mg |

Beispiel 12

Aerosol-Spray mit 1 µg (-)-Cimaterol pro Hub

Zusammensetzung:

| Wirksubstanz | 0,00025 mg |
|---|---|
| Ethanol rein 99,9%ig | 0,87500 mg |
| Treibgasgemisch 11/12/114 (23:54:23) | 69,12475 mg |
| | 70,00000 mg |

Beispiel 13

Inhalationslösung mit 59 mg (-)-Cimaterol pro 100 ml

Zusammensetzung:

| Wirksubstanz | 0,59 mg |
|---|---|
| Natriumchlorid | 900,00 mg |
| Benzalkoniumchlorid | 25,00 mg |
| Dest. Wasser ad | 100,00 ml |

Herstellungsverfahren:

Die Wirksubstanz, Kochsalz und Benzalkoniumchlorid werden in dest. Wasser gelöst und anschließend keimfrei filtriert.

Beispiel 14

Alleinfutter II für Mastschweine

| Gerste | 379 g/kg |
|---|---|
| Weizennachmehl | 200 g/kg |
| Maniokmehl | 135 g/kg |
| Ackerbohnen | 100 g/kg |
| Raps-Extraktionsschrot | 100 g/kg |
| Futterfett | 65 g/kg |
| lysinreiches Mineralfutter für Schweine | 20 g/kg |
| Wirkstoff-Vormischung | 1 g/kg |

Diese Komponenten in den angegebenen Mengen ergeben nach sorgfältigem Mischen 1 kg Futter. Die 1 g Wirkstoffvormischung enthalten z.B. 2 mg Wirkstoff und 0,998 g Maisstärke.

Beispiel 15

Mastfutter II für Broiler

| Mais | 634 g/kg |
|---|---|
| Sojabohnenmehl | 260 g/kg |
| Fleischmehl | 40 g/kg |
| Futterfett | 25 g/kg |
| Sojaöl | 17 g/kg |
| Bicalciumphosphat | 12 g/kg |
| Calciumcarbonat | 6 g/kg |
| Vitamin-/Mineralstoffmischung | 5 g/kg |
| Wirkstoff-Vormischung | 1 g/kg |

Diese Komponenten in den angegebenen Mengen ergeben nach sorgfältigem Mischen 1 kg Futter. Die 1 g Wirkstoffvormischung enthalten z.B. 1 mg Wirkstoff und 0,999 g Maisstärke.

Beispiel 16

Kraftfutter für Rinder

| | |
|---|---|
| Zuckerrübenschnitzel | 600,0 g/kg |
| Maiskleber | 100,0 g/kg |
| Malzkeime | 50,0 g/kg |
| Sojabohnenmehl | 35,0 g/kg |
| Weizen | 119,0 g/kg |
| Melasse | 60,0 g/kg |
| Futterphosphate | 12,0 g/kg |
| Calciumcarbonat | 2,5 g/kg |
| Salz | 5,0 g/kg |
| Mineralstoffe | 10,0 g/kg |
| Vitamin-Vormischung | 5,5 g/kg |
| Wirkstoff-Vormischung | 1,0 g/kg |

Diese Komponenten in den angegebenen Mengen ergeben nach sorgfältigem Mischen 1 kg Futter.
Die 1 g Wirkstoffvormischung enthalten z.B. 2 mg Wirkstoff und 0,998 g Maisstärke.

Beispiel 17

Mastfutter für Lämmer

| | |
|---|---|
| Gerste | 690 g/kg |
| Sojabohnenmehl | 100 g/kg |
| Mais | 159 g/kg |
| Melasse | 30 g/kg |
| Vitamin-/Mineralstoffmischung | 20 g/kg |
| Wirkstoffvormischung | 1 g/kg |

Diese Komponenten in den angegebenen Mengen ergeben nach sorgfältigem Mischen 1 kg Futter.
Die 1 g Wirkstoffvormischung enthalten z.B. 2 mg Wirkstoff und 0,998 g Maisstärke.

**Patentansprüche**

1.  Im wesentlichen optisch reines (-)-4-Amino-1-(-3'-cyanophenyl)-2-isopropylamino-ethanol und dessen Säureadditionssalze.

2.  Verbindung nach Anspruch 1, dadurch gekennzeichnet, daß sie eine optische Reinheit von mindestens ee = 90 % aufweist.

3.  Verbindung nach Anspruch 1, dadurch gekennzeichnet, daß sie eine optische Reinheit von mindestens ee = 94 % aufweist.

**4.** Physiologisch verträgliche Säureadditionssalze der Verbindung nach mindestens einem der Ansprüche 1 bis 3.

**5.** Arzneimittel, enthaltend eine Verbindung nach mindestens einem der Ansprüche 1 bis 3 oder ein physiologisch verträgliches Säureadditionssalz gemäß Anspruch 4 neben gegebenenfalls einem oder mehreren inerten Trägerstoffen und/oder Verdünnungsmitteln.

**6.** Verwendung einer Verbindung nach mindestens einem der Ansprüche 1 bis 3 oder ein physiologisch verträgliches Säureadditionssalz gemäß Anspruch 4 zur Herstellung eines Arzneimittels, das für die Behandlung der Fettsucht, von obstruktiven Lungenveränderungen, allergischem Asthma-Bronchiale, spastischen Bronchitiden, Entzündungen oder vorzeitiger Wehentätigkeit geeignet ist.

**7.** Verfahren zur Herstellung eines Arzneimittels gemäß Anspruch 5, dadurch gekennzeichnet, daß auf nichtchemischem Wege eine Verbindung nach mindestens einem der Ansprüche 1 bis 3 oder ein physiologisch verträgliches Säureadditionssalz gemäß Anspruch 4 in einen oder mehrere inerten Trägerstoffen und/oder Verdünnungsmitteln eingearbeitet wird.

**8.** Verwendung einer Verbindung nach mindestens einem der Ansprüche 1 bis 3 oder ein physiologisch verträgliches Säureadditionssalz gemäß Anspruch 4 als leistungsförderndes Mittel bei Tieren.

**9.** Tiernahrung und Prämixe zur Herstellung von Tiernahrung, enthaltend eine Verbindung nach mindestens einem der Ansprüche 1 bis 3 oder ein physiologisch verträgliches Säureadditionssalz gemäß Anspruch 4.

**10.** Leistungsförderndes Mittel für Tiere, enthaltend eine Verbindung nach mindestens einem der Ansprüche 1 bis 3 oder ein physiologisch verträgliches Säureadditionssalz gemäß Anspruch 4.

**11.** Verfahren zur Herstellung von leistungsfördernden Mitteln für Tiere, dadurch gekennzeichnet, daß eine Verbindung nach mindestens einem der Ansprüche 1 bis 3 oder ein physiologisch verträgliches Säureadditionssalz gemäß Anspruch 4 mit Streck-, Verdünnungs- und Nahrungsmitteln sowie gegebenenfalls weiteren Hilfsstoffen vermischt wird.

## Claims

**1.** Substantially optically pure (-)-1-(4'-amino-3'-cyano-phenyl)-2-isopropylamino-ethanol and the acid addition salts thereof.

**2.** Compound according to claim 1, characterized in that it has an optical purity of at least ee = 90%.

**3.** Compound according to claim 1, characterised in that it has an optical purity of at least ee = 94%.

**4.** Physiologically acceptable acid addition salts of the compound according to at least one of claims 1 to 3.

**5.** Pharmaceutical composition containing a compound according to at least one of claims 1 to 3 or a physiologically acceptable acid addition salt according to claim 4 optionally together with one or more inert carriers and/or diluents.

**6.** Use of a compound according to at least one of claims 1 to 3 or a physiologically acceptable acid addition salt according to claim 4 for preparing a pharmaceutical composition which is suitable for treating obesity, obstructive lung changes, allergic bronchial asthma, spastic bronchitis, inflammation or premature labour.

**7.** Process for preparing a pharmaceutical composition according to claim 5, characterized in that a compound according to at least one of claims 1 to 3 or a physiologically acceptable acid addition salt according to claim 4 is incorporated in one or more inert carriers and/or diluents by a non-chemical method.

**8.** Use of a compound according to at least one of claims 1 to 3 or a physiologically acceptable acid addition salt according to claim 4 as a performance enhancing agent in animals.

**9.** Animal feed and premixes for producing animal feed, containing a compound according to at least one of claims 1 to 3 or a physiologically acceptable acid addition salt according to claim 4.

**10.** Performance enhancing agent for animals, containing a compound according to at least one of claims 1 to 3 or a physiologically acceptable acid addition salt according to claim 4.

**11.** Process for preparing performance enhancing agents for animals, characterized in that a compound according to at least one of claims 1 to 3 or a physiologically acceptable acid addition salt according to claim 4 is mixed with extenders, diluents and nutrients and optionally other adjuvants.

**Revendications**

**1.** (-)-4-amino-1-(3'-cyanophényl)-2-isopropylaminoéthanol sensiblement pur optiquement et ses sels d'addition d'acide.

**2.** Composé selon la revendication 1 caractérisé en ce qu'il présente une pureté optique d'au moins e.e. = 90%.

**3.** Composé selon la revendication 1 caractérisé en ce qu'il présente une pureté optique d'au moins e.e. = 94%.

**4.** Sels d'addition d'acide physiologiquement acceptables du composé selon au moins l'une des revendications 1 à 3.

**5.** Médicament contenant un composé selon au moins l'une des revendications 1 à 3 ou un sel d'addition d'acide physiologiquement acceptable selon la revendication 4 et éventuellement un ou plusieurs supports et/ou diluants inertes.

**6.** Utilisation d'un composé selon au moins l'une des revendications 1 à 3 ou d'un sel d'addition d'acide physiologiquement acceptable selon la revendication 4 pour la préparation d'un médicament qui est approprié au traitement de l'adipose, des modifications pulmonaires obstructives, de l'asthme bronchique allergique, des bronchites spastiques, des inflammations ou de l'activité prématurée des douleurs de l'accouchement.

**7.** Procédé de préparation d'un médicament selon la revendication 5 caractérisé en ce que, par voie non chimique, un composé selon au moins l'une des revendications 1 à 3 ou un sel d'addition d'acide physiologiquement acceptable selon la revendication 4 est incorporé dans un ou plusieurs supports et/ou diluants inertes.

**8.** Utilisation d'un composé selon au moins l'une des revendications 1 à 3 ou d'un sel d'addition d'acide physiologiquement acceptable selon la revendication 4 comme agent favorisant les performances chez les animaux.

**9.** Nourriture pour animaux et prémélanges pour la préparation de nourriture pour animaux, contenant un composé selon au moins l'une des revendications 1 à 3 ou un sel d'addition d'acide physiologiquement acceptable selon la revendication 4.

**10.** Agent favorisant les performances pour animaux contenant un composé selon au moins l'une des revendications 1 à 3 ou un sel d'addition d'acide physiologiquement acceptable selon la revendication 4.

**11.** Procédé de préparation d'agents favorisant les performances pour animaux caractérisé en ce qu'un composé selon au moins l'une des revendications 1 à 3 ou un sel d'addition d'acide physiologiquement acceptable selon la revendication 4 est mélangé avec des charges, des diluants et des aliments, et éventuellement d'autres adjuvants.